# EUROPÄISCHE PATENTSCHRIFT

(11) **EP 0 088 729 B1**
(45) Veröffentlichungstag und Bekanntmachung des Hinweises auf die Patenterteilung: **29.07.1987**
(21) Anmeldenummer: 83810087.3
(22) Anmeldetag: 03.03.1983
(51) Int. Cl.: C08K 5/30, C07C 109/12, C09B 57/10

(54) **Verwendung von Metallkomplexen von Hydrazonen als Pigmente**
Use of metal complexes of hydrazones as pigments
Utilisation de complexes métalliques d'hydrazones comme pigments

(30) Priorität: 09.03.1982 CH 1438/82
(43) Veröffentlichungstag der Anmeldung: 14.09.1983
(73) Patentinhaber: CIBA-GEIGY AG, 4002 Basel (CH)
(72) Erfinder: Felix, Franz, Dr., CH-4142 Münchenstein (CH)

## Beschreibung

Die GB-PS 1512679 beschreibt ein Verfahren zum Pigmentieren von hochmolekularem organischem Material durch die Verwendung von Metallkomplexen von Hydrazonen der Formel (0) 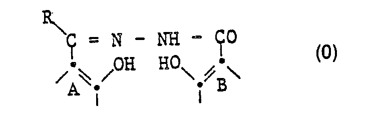 worin A und B isocyclische oder heterocyclische aromatische Reste sind, wobei nur einer der Reste A und B ein Benzolring ist, und R Wasserstoff, C₁-C₆-Alkyl oder Aryl bedeutet. Die Verwendung dieser Pigmente ergibt aber Ausfärbungen, die den Anforderungen an die Farbstärke, Nuancenreinheit, Brillanz und Echtheiten nicht genügen.

Die Erfindung betrifft ein Verfahren zum Pigmentieren von hochmolekularem organischem Material, dadurch gekennzeichnet, dass man einen Uebergangsmetall-Komplex eines Hydrazons der Formel I verwendet 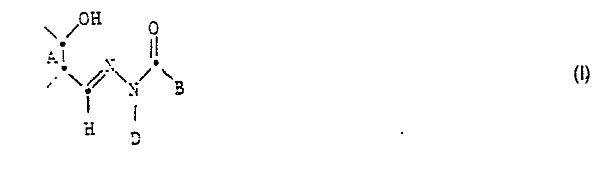 worin A einen isocyclischen oder heterocyclischen aromatischen Rest und B unsubstituiertes Phenyl oder Naphthyl oder durch ein bis zwei gleiche oder verschiedene Substituenten aus der Gruppe bestehend aus C₁-C₃ Alkyl, C₁-C₃ Alkoxy, Chlor, Brom, Nitro, Phenyl, Phenoxy, Chlorphenoxy, Bromphenoxy, Methylphenoxy, Methoxyphenoxy, Benzoylamino, Methylbenzoylamino, Methoxybenzoylamino, Chlorbenzoylamino und Brombenzoylamino substituiertem Phenyl oder Naphthyl bedeuten, und D Wasserstoff bedeutet, wobei, falls B ein Phenyl oder Naphthyl ist, D Wasserstoff oder eine Carbonylgruppe darstellt, die zusammen mit dem Strukturelement 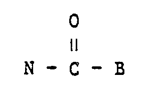 einen Phthalimid- oder Naphthalimidrest bildet.

A kann als isocyclischer oder heterocyclischer aromatischer Rest insbesondere ein unsubstituierter oder ein ein- bis zweifach substituierter Benzol-, Naphthalin-, Pyridin-, Pyrimidin-, Pyrazolon-, Chinolin-, Isochinolon-, Pyrazol- oder Cumarinrest sein.

Mögliche Substituenten sind beispielsweise Halogen, C₁-C₆-Alkyl, C₁-C₆-Alkoxy, Cyano, Nitro, Carboxy, C₂-C₇-Alkoxycarbonyl, Carbamoyl, unsubstituiertes Phenyl, Phenoxy, Phenylcarbamoyl, Benzoylamino, durch Halogen, C₁-C₆-Alkyl oder C₁-C₆-Alkoxy substituiertes Phenyl, Phenoxy, Phenylcarbamoyl oder Benzoylamino.

Halogen kann beispielsweise Fluor, Chlor oder Brom, vorzugsweise Chlor oder Brom bedeuten.

Cₗ-C₆-Alkyi kann geradkettig oder verzweigt sein und insbesondere Methyl, Aethyl, Isopropyl, sec.-Butyl, tert.-Butyl, tert.-Amyl oder n-Hexyl sein. C₁-C₃-Alkyl-Substituenten von B als substituierter Phenyl-oder Naphthylrest sind Methyl, Aethyl, n-Propyl oder Isopropyl.

C₁-C₆-Alkoxy kann geradkettig oder verzweigt sein und insbesondere Methoxy, Aethoxy, Propoxy, Isopropoxy, n-Butoxy, n-Pentoxy oder n-Hexyloxy sein. C₁-C₃-Alkoxy-Substituenten von B als substituierter Phenyl- oder Naphthylrest sind Methoxy, Aethoxy, n-Propoxy oder Isopropoxy.

C₂-C₇-Alkoxycarbonyl kann geradkettig oder verzweigt sein und insbesondere Methoxycarbonyl, Aethoxycarbonyl, n-Propoxycarbonyl, lsopropoxycarbonyl, tert.-Butoxycarbonyl, n-Pentoxycarbonyl oder n-Hexyloxycarbonyl sein.

Bedeuten die Substituenten an A und B substituiertes Phenyl, Phenoxy, Phenylcarbamoyl oder Benzoylamino, so kann es sich um 4-Chlorphenyl, 2,4-Dichlorphenyl, 4-Methylphenylcarbamoyl, 3,4-Dichlorphenylcarbamoyl, 4-Methoxyphenylcarbamoyl, 4-Methylbenzoylamino, 4-Chlorbenzoylamino, 3-Chlor-4-methylbenzoylamino, oder 4-Methoxybenzoylamino handeln.

Als Uebergangsmetalle kommen insbesondere zweifach positiv geladene lonen, wie Ni²⁺, Cu²⁺, Zn²⁺ und CO²⁺, vor allem aber Ni²⁺ und Cu²⁺ in Frage.

Auf 1 Metallion können 1 oder 2 Reste eines Hydrazons der Formel I entfallen, so dass 1 : 1- oder 1 2-Metallkomplexe vorliegen können. Im erfindungsgemässen Verfahren verwendet man aber bevorzugt 1 : 1-Kupferkomplexe oder insbesondere 1 : 2-Nickelkomplexe eines Hydrazons der Formel I.

Die 1 : 1-Metallkomplexe können monomer oder, besonders jene des Cu²⁺, dimer sein. Bei den monomeren 1 : 1-Metallkomplexen kann die vierte Koordinationsstelle des Metallions durch einen zusätzlichen Liganden, wie H₂0, NH₃ oder CH₃COO^{s} abgesättigt sein. Dies hängt weitgehend von den Herstellungsbedingungen der Metallkomplexe und der Natur des eingesetzten metallabgebenden Mittels ab. Monomere 1 : 1-Metallkomplexe mit Acetatresten können durch folgende Formel dargestellt werden 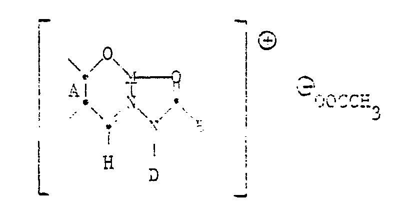 worin M das Metallatom und die Symbole A, B und D die oben genannte Bedeutung haben.

Im erfindungsgemässen Verfahren verwendet man bevorzugt einen 1 : 1- oder 1 : 2-Nickelkomplex oder 1 : 1-Kupferkomplex, vor allem aber einen 1 : 2-Nickel- oder 1 : 1-Kupferkomplex eines Hydrazons der Formel II 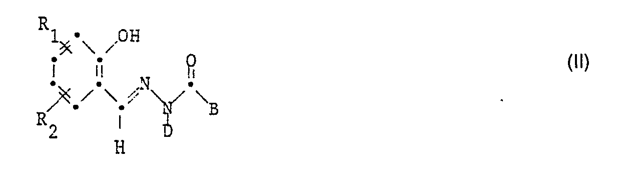 oder der Formel III 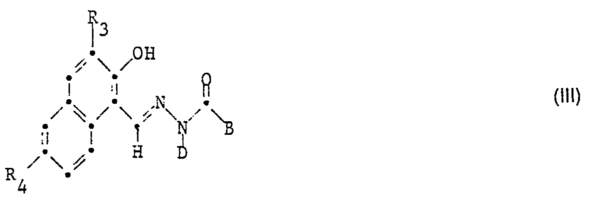 worin R₁ und R₂ unabhängig voneinander Wasserstoff, Chlor, Brom, C₁-C₃-Alkyl, C₁-C₃-Alkoxy, Cyano oder Nitro bedeuten, und R₃ Wasserstoff, Carboxy, C₂-C₄-Alkoxycarbonyl, Carbamoyl, unsubstituiertes oder durch C₁-C₃-Alkyl, C₁-C₃-Alkoxy, Chlor oder Brom einfach substituiertes Phenylcarbamoyl bedeutet, und R₄ Wasserstoff, Chlor, Brom oder C₁-C₃-Alkoxy bedeutet, und B unsubstituiertes Phenyl oder Naphthyl oder durch ein bis zwei gleiche oder verschiedene Substituenten aus der Gruppe bestehend aus C₁-C₃-Alkyl, C₁-C₃-Alkoxy, Chlor, Brom, Nitro, Phenyl, Phenoxy, Chlorphenoxy, Bromphenoxy, Methylphenoxy, Methoxyphenoxy, Benzoylamino, Methylbenzoylamino, Methoxybenzoylamino, Chlorbenzoylamino und Brombenzoylamino substituiertem Phenyl oder Naphthyl bedeutet, und D Wasserstoff oder eine Carbonylgruppe darstellt, die zusammen mit dem Strukturelement 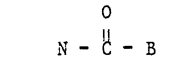 einen Phthalimid- oder Naphthalimidrest bildet.

Mit besonderem Interesse verwendet man im erfindungsgemässen Verfahren den 1 : 1- oder 1 : 2-Nickelkomplex oder 1 : 1-Kupferkomplex eines Hydrazones der Formel II oder III, worin R₁ und R₂ unabhängig voneinander Wasserstoff, Chlor oder Brom sind, und R₃ Wasserstoff, Carboxy, Methoxycarbonyl, Carbamoyl oder Phenylcarbamoyl bedeutet, und R₄ Wasserstoff oder Brom bedeutet, und B unsubstituiertes Phenyl oder Naphthyl oder durch ein bis zwei gleiche oder verschiedene Substituenten aus der Gruppe bestehend aus Methyl. Methoxy, Chlor, Brom, Nitro oder Phenyl substituiertes Phenyl oder Naphthyl bedeutet, und D Wasserstoff ist.

Im erfindungsgemässen Verfahren verwendet man besonders bevorzugt den 1 : 1- oder 1 : 2-Nickelkomplex oder 1 : 1-Kupferkomplex eines Hydrazons der Formel III, worin R₃ Wasserstoff, Carboxy oder Phenylcarbamoyl bedeutet und R₄ Wasserstoff oder Brom ist, und D Wasserstoff und B unsubstituiertes oder durch Chlor, Brom, Methyl, Methoxy oder Nitro einfach substituiertes Phenyl bedeuten.

Besonders bevorzugt verwendet man den 1 : 1- oder 1 : 2-Nickelkomplex oder 1 : 1-Kupferkomplex eines Hydrazones der Formel II, worin R₁ und R₂ Chlor sind, B durch Methyl substituiertes Phenyl bedeutet, und D Wasserstoff ist.

Mit ganz besonderem Interesse verwendet man den 1 : 1- oder 1 : 2-Nickelkomplex oder 1 : 1-Kupferkomplex eines Hydrazones der Formel III, worin R₃, R₄ und D Wasserstoff bedeuten und B unsubstituiertes Phenyl ist.

Die im erfindungsgemässen Verfahren verwendeten Verbindungen sind neu, soweit es sich um 1 : 1-oder 1 : 2-Nickelkomplexe oder 1 : 1-Kupferkomplexe von Hydrazonen der Formel I, worin A einen heterocyclischen aromatischen Rest darstellt, und die Symbole B und D die oben angegebene Bedeutung haben, und von Hydrazonen der Formel 11 und III handelt, worin R₁ und R₂ beide entweder Chlor oder Brom sind, R₃ Wasserstoff, Carboxy, C₂-C₇Alkoxycarbonyl, Carbamoyl oder Phenylcarbamoyl bedeutet, R₄ Wasserstoff oder Brom ist, B unsubstituiertes Phenyl oder Naphthyl oder durch C₁-C₆-Alkyl, C₁-C₆-Alkoxy, Halogen, Nitro, Phenyl, Phenoxy, Halogenphenoxy, C₇-C₁₂-Alkylphenoxy, C₇-C₁₂-Alkoxypheno- xy, Benzoylamino, C₈-C₁₃-Alkylbenzoylamino, C₈-C₁₃-Alkoxybenzoylamino, Halogenbenzoylamino substituiertes Phenyl oder Naphthyl bedeutet, und D Wasserstoff oder eine Carbonylgruppe ist, die zusammen mit dem Strukturelement 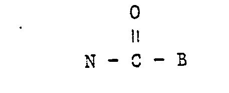 einen Phthalimid- oder Naphthalimidrest bildet, wobei R₃ oder R₄ verschieden von Wasserstoff sein muss, wenn D Wasserstoff ist und B unsubstituiertes Phenyl bedeutet.

Sowohl die bekannten wie die neuen Uebergangsmetallkomplexe der Hydrazone der Formel I können - hergestellt werden, indem man eine Verbindung der Formel IV 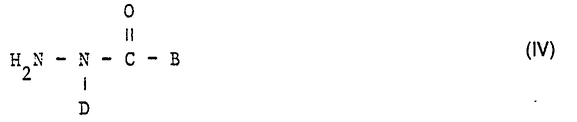 worin D und B die oben angegebene Bedeutung haben, mit einem Aldehyd der Formel V 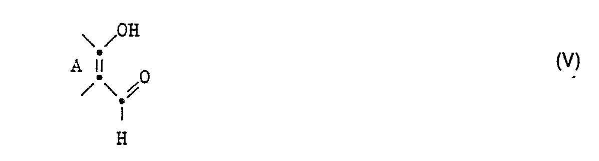 oder einem Aldimin der Formel VI 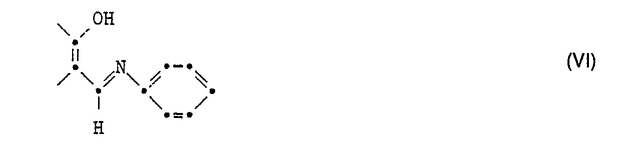 worin A die oben angegebene Bedeutung hat, in einem Lösungsmittel kondensiert und während oder nach der Kondensation ein metallabgebendes Mittel zusetzt.

Nach diesem Verfahren können sowohl die 1 : 1- wie die 1 : 2-Metallkomplexe hergestellt werden. In der Regel fördern elektronenanziehende Gruppen in den Resten A oder B und ein Ueberschuss an metallabgebendem Mittel die Bildung des 1 : 1-Metallkomplexes.

Als metallabgebende Mittel eignen sich insbesondere M-Formiat, M-Stearat, M-Acetylacetonat, vor allem M-Acetat, wobei M das Uebergangsmetall bedeutet.

Als Lösungsmittel verwendet man ein organisches Lösungsmittel, insbesondere aber Wasser. Es können auch Mischungen von Lösungsmitteln eingesetzt werden. Die Verwendung von Wasser als Lösungsmittel ist neu und stellt somit ebenfalls einen Gegenstand der Erfindung dar.

Als organische Lösungsmittel eignen sich insbesondere Aethanol, Butanol, Essigsäure, Dioxan, Dimethylformamid, Aethylcellosolve, Aethylenglykolmonomethyläther oder N-Methylpyrrolidon.

Kondensation und Behandlung mit dem metallabgebenden Mittel erfolgen bevorzugt bei erhöhter Temperatur, insbesondere zwischen 50°C, vor allem 80 °C und dem Siedepunkt des verwendeten Lösungsmittels.

Die Metallkomplexe der Hydrazone der Formel I lassen sich leicht durch Filtration isolieren. Allfällige Verunreinigungen können durch Auswaschen entfernt werden.

Die Verbindungen der Formel IV, V und VI sind bekannt und lassen sich nach bekannten Verfahren herstellen.

Hochmolekulare organische Materialien, die mit den Uebergangsmetallkomplexen von Hydrazonen der Formel I pigmentiert werden können, sind z. B. Celluloseäther und -ester, wie Aethylcellulose, Nitrocellulose, Celluloseacetat, Cellulosebutyrat, natürliche Harze oder Kunstharze, wie Polymerisationsharze oder Kondensationsharze, z. B. Aminoplaste, insbesondere Harnstoff- und Melamin-Formaldehydharze, Alkydharze, Phenoplaste, Polycarbonate, Polyolefine, wie Polystyrol, Polyvinylchlorid, Polyäthylen, Polypropylen, Polyacrylnitril, Polyacrylsäureester, Polyamide, Polyurethane oder Polyester, Gummi, Casein, Silikon und Silikonharze, einzeln oder in Mischungen.

Dabei spielt es keine Rolle, ob die erwähnten hochmolekularen Verbindungen als plastische Massen. Schmelzen oder in Form von Spinnlösungen, Lacken, Anstrichstoffen oder Druckfarben vorliegen. Je nach Verwendungszweck erweist es sich als vorteilhaft, die erfindungsgemäss zu verwendenden Pigmente als Toner oder in Form von Präparaten einzusetzen. Bezogen auf das zu pigmentierende hochmolekulare organische Material setzt man den Metallkomplex des Hydrazons der Formel I in einer Menge von vorzugsweise 0,1 bis 10 Gew.-% ein.

Die Pigmente können in der Form, wie sie in der Synthese anfallen, verwendet werden. In leicht gemahlener Form liefern sie opake Ausfärbungen. Man kann sie aber auch einer intensiveren Mahlung unterziehen und erhält dann transparente Pigmente, die sich zur Herstellung von farbstarken Metalleffektlackierungen besonders gut eignen.

Werden die erfindungsgemäss zu verwendenden Pigmente in Kunststoffe oder Fasern eingearbeitet, so stabilisieren sie diese gegen die Einflüsse von Licht und Wetter. Aus gefärbten Substraten hergestellte Gegenstände zeichnen sich durch eine längere Gebrauchsdauer aus.

Werden die erfindungsgemäss zu verwendenden Pigmente in lichtstabilisiertes hochmolekulares organisches Material eingearbeitet, wird in der Regel dessen Lichtstabilität nicht beeinträchtigt.

Gegebenenfalls lässt sich auch eine synergistische Verbesserung der Lichtstabilität beobachten.

Die erhaltenen Färbungen zeichnen sich durch gute allgemeine Pigmenteigenschaften, insbesondere durch ausgezeichnete Licht-, Hitze-, Wetter-, Ueberlackier- und Migrationsechtheit und durch für Metallkomplexe unerwartet hohe Brillanz des Farbtons, hohe Farbstärke und gute Dispergierbarkeit aus. Die Pigmente beeinflussen die mechanischen Eigenschaften der Substrate wenig, insbesondere fördern sie die Verzugseigenschaften von Kunststoffteilen nicht. Pigmentierte Fasern zeigen allgemein ein hohes Echtheitsniveau.

Die folgenden Beispiele erläutern die Erfindung. Die Teile bedeuten Gewichtsteile und die Prozente Gewichtsprozente.

### Beispiel 1

65 Teile stabilisiertes Polyvinylchlorid, 35 Teile Dioctylphthalat und 0,2 Teile feinverteilter 1 : 2-Nickelkomplex aus dem Benzoylhydrazon der Formel VII 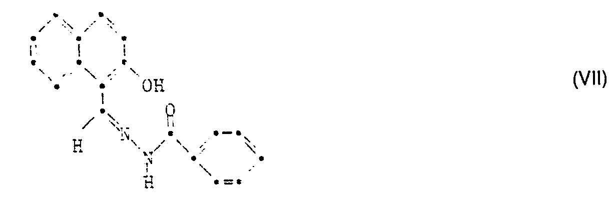 werden miteinander verrührt und auf einem Zweiwalzenkalander 7 Minuten bei 160 °C gewalzt. Man erhält eine gelb gefärbte Folie von sehr guter Licht- und Migrationsechtheit.

Herstellung der feinverteilten Form des 1 : 2-Nickelkomplexes : 25 Teile 1 : 2-Nickelkomplex. 100 Teile fein gemahlenes Natriumchlorid und 30 Teile Diacetonalkohol werden zusammen während 5 Stunden unter Kühlung in einem Laboratoriumskneter geknetet. Man gibt die Mischung in 4000 Volumenteile Wasser und filtriert die entstandene Suspension ab. Man wäscht das isolierte Pigment mit Wasser und trocknet es im Vakuum bei 80 °C.

### Beispiel 2

10 g Titandioxyd und 2 g 1 : 2-Nickelkomplex aus dem Benzoylhydrazon der Formel VII werden mit 88 g einer Mischung von 26,4 g Kokosalkydharz, 24,0 g Melamin-Formaldehydharz (50 % Festkörpergehalt), 8,8 g Aethylenglykolmonomethyläther und 28,8 g Xylol während 48 Stunden in einer Kugelmühle vermahlen. Wird dieser Lack auf eine Aluminiumfolie gespritzt, 30 Minuten bei Raumtemperatur vorgetrocknet und dann während 30 Minuten bei 120 °C eingebrannt, dann erhält man eine gelbe Lackierung, die sich bei guter Farbstärke durch eine sehr gute Ueberlackier-, Licht- und Wetterechtheit auszeichnet.

### Beispiel 3

4 Teile des fein verteilten Pigments gemäss Beispiel 1 werden in 20 Teilen Lösungsmittel der folgenden Zusammensetzung eingerührt: 50 Teile Solvesso 150® (Gemisch aromatischer Kohlenwasserstoffe), 15 Teile Butylacetat, 5 Teile Exkin II® (Verlaufmittel auf Ketoximbasis), 25 Teile Methylisobutylketon, 5 Teile Silikonöl (1 % in Solvesso 150®). Wenn die vollständige Feinverteilung erreicht ist (je nach Art des Rührens in ca. 15-60 Minuten), werden die Bindemittel zugesetzt, nämlich 48,3 Teile Baycryl L 530^{@} (Acrylharz) (51 % in Xylol/Butanol 3 1) und 23,7 Teile Maprenal TTX" (Melaminharz) (55 % in Butanol). Nach kurzem Homogenisieren wird der Lack nach üblichen Methoden wie Spritzen und Tauchen oder speziell zur kontinuierlichen Beschichtung von Metallblechen im « Coil-Coating »-Verfahren appliziert und eingebrannt (Einbrennen 30 Minuten, 130 °C). Die erhaltenen gelben Lackierungen zeichnen sich aus durch sehr guten Verlauf, hohen Glanz und gute Feinverteilung des Pigmentes, sowie durch gute Wetterechtheiten.

### Beispiel 4

2 g 1 : 2-Nickelkomplex aus dem Benzoylhydrazon der Formel VII werden mit 36 g Tonerdehydrat, 60 g Leinölfirnis von mittlerer Viskosität und 2 g Kobaltlinoleat auf dem Dreiwalzenstuhl angerieben. Die mit der entstehenden Farbpaste erzeugten gelben Drucke sind farbstark und gut lichtecht.

### Beispiel 5

Ein zur Faserherstellung geeignetes Polypropylengranulat wird mit 2,5 % eines Pigmentpräparates. enthaltend 40 % 1 : 2-Nickelkomplex aus dem Benzoylhydrazon der Formel VII, gründlich gemischt. Die Mischung wird auf einer Schmelzspinnanlage bei 240-260°C zu Fäden versponnen, die anschliessend auf einer Streckzwirnanlage im Verhältnis 1 : 4 verstreckt und aufgespult werden. Man erhält eine kräftige gelbe Färbung, die sich durch gute Licht-, Wäsche-, Trockenreinigungs-, Abgas- und Peroxydbleichechtheiten auszeichnet. Bei sonst analoger Arbeitsweise erhält man ebenfalls sehr echte, gelbe Ausfärbungen, wenn man anstelle von Polypropylen- Polycaprolactamgranulat verwendet, und die Mischung bei 260-290 °C zu Fäden verspinnt.

Das oben eingesetzte Präparat wird wie folgt hergestellt :
40 Teile des gelben Pigmentes, 60 Teile Mg-Behenat und 500 Teile Natriumchlorid werden in einem Pulvermischer gründlich gemischt. Diese Mischung wird mit einer Laborknetmaschine bei 130 °C bearbeitet. Das erhaltene Präparat wird mit Wasser vermahlen, filtriert, salzfrei gewaschen, getrocknet und pulverisiert.

### Beispiele 6 bis 35

Ebenfalls echte gelbe Ausfärbungen in Kunststoffen, Lacken, Fasern oder Druckfarben erhält man, wenn man analog zu den Beispielen 1 bis 5 arbeitet, jedoch anstelle des dort verwendeten Pigmentes einen gelben 1 : 2-Nickelkomplex eines in Tabelle 1 und 2 aufgeführten Hydrazons einsetzt :
(Siehe Tabellen Seite 7 ff.)

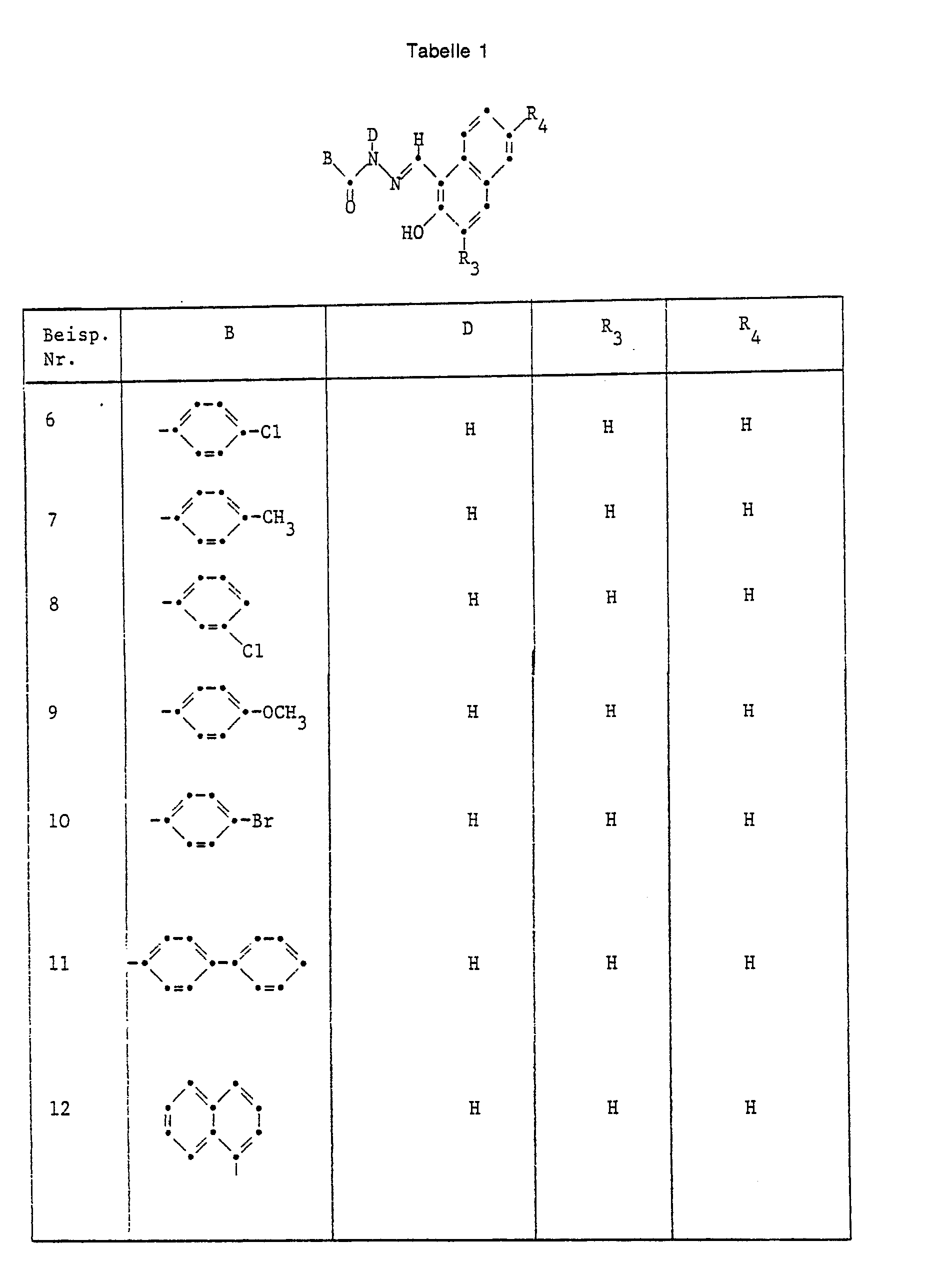

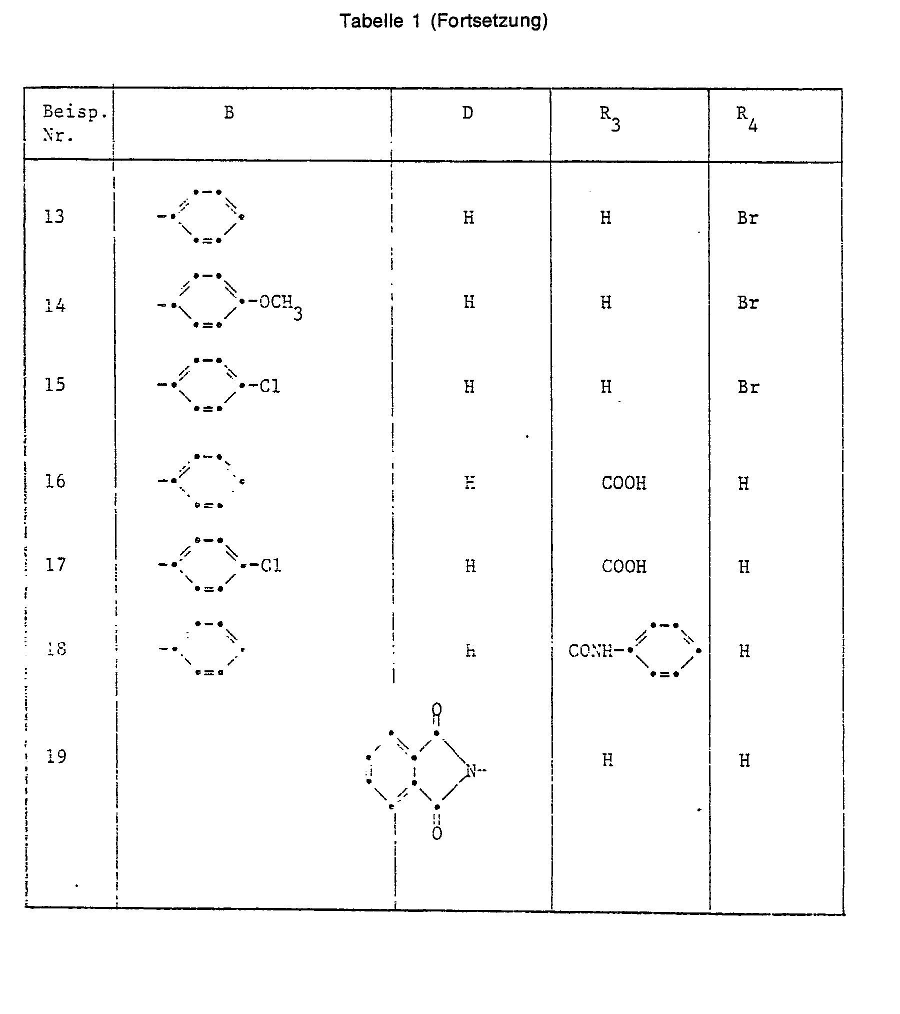

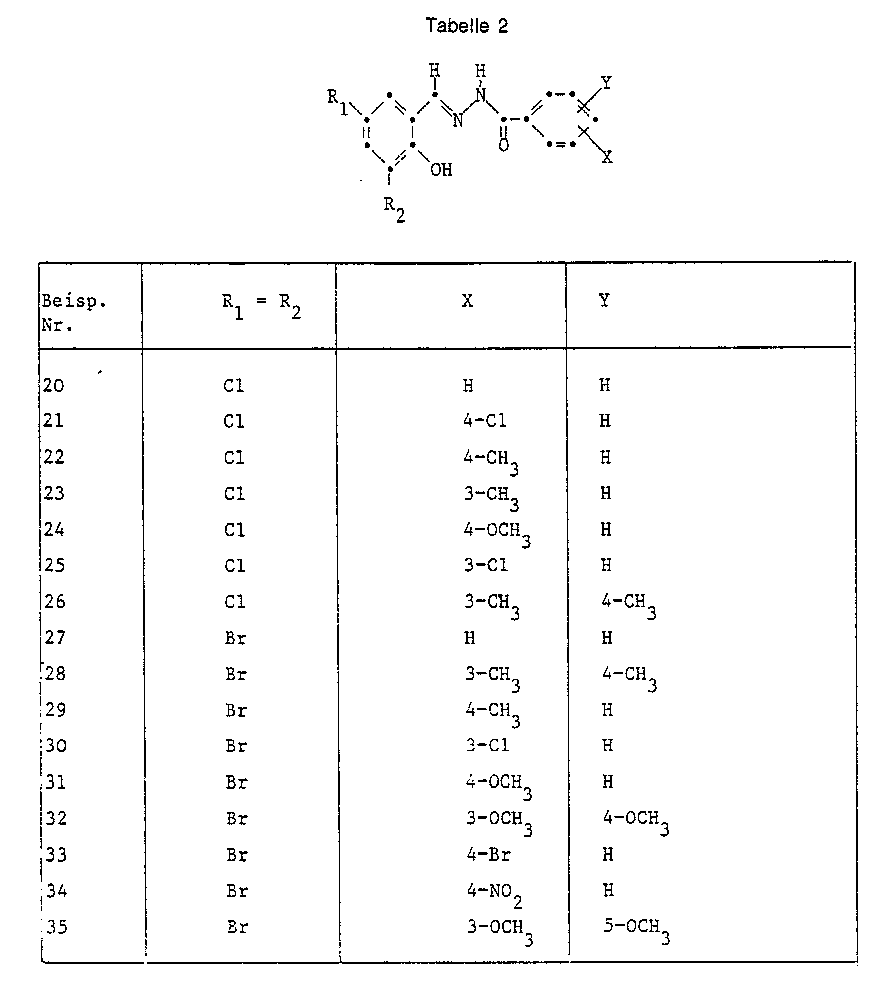

### Beispiele 36 und 37

Echt gelbe Ausfärbungen in Kunststoffen, Lacken, Fasern oder Druckfarben erhält man ferner, wenn man analog den Beispielen 1 bis 5 arbeitet, jedoch anstelle des dort verwendeten Pigmentes einen 1 : 2-Nickelkomplex aus einem Hydrazon der Formel 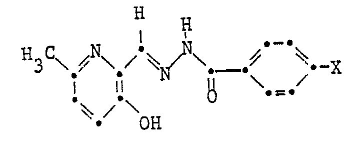 verwendet, worin X für Wasserstoff bzw. Chlor steht.

### Beispiel 38

5.1 g 2-Hydroxy-6-brom-1-naphthaldehyd und 3.4 g 4-Chlorbenzoesäurehydrazid werden in 60 ml Essigsäure während 1 Stunde bei 90 °C gerührt. Dann gibt man 2.6 g Nickelacetattetrahydrat zu und erhöht die Temperatur auf 115°C. Nach 3 Stunden Rühren wird heiss filtriert und mit Alkohol gewaschen. Nach dem Trocknen im Vakuum bei 60 °C erhält man 3.7 g eines gelben 1 : 2-Nickelkomplexes des Hydrazons der Formel

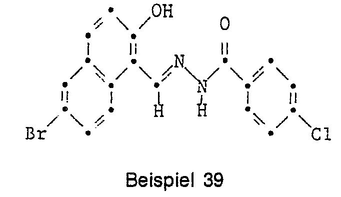

6.9 g 2-Hydroxy-1-naphthaldehyd und 6.8 g 3-Chlorbenzhydrazid werden in 50 ml Aethylenglykolmonoäthyläther während 45 Minuten bei 90 °C gerührt. Dann gibt man 5.2 g Nickelacetylacetonat zu und rührt drei Stunden bei 115 °C. Die Suspension wird heiss filtriert, mit Aethanol gewaschen und bei 60 °C im Vakuum getrocknet: Man erhält 11.9 g eines gelben 1 : 2-Nickelkomplexes des Hydrazons der Formel

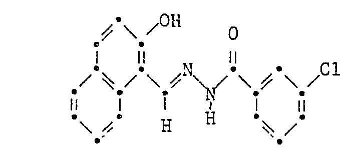

Mikroanalyse :
Berechnet: C 61.4 H 3.15 N 7.9 Ni 8.3 %
Gefunden : C 60.7 H 3.6 N 7.8 Ni 8.1 %.

### Beispiel 40

5.6 g 3,5-Dibromsalicylaldehyd und 3.2 g 3,4-Dimethylbenzhydrazid werden in 60 ml Butanol zusammengegeben, auf 90 °C erwärmt und 30 Minuten gerührt. Dann gibt man 2.7 g Nickelacetylacetonat zu und rührt die Mischung 2 Stunden bei 110 °C. Man filtriert bei 80 °C, wäscht den Filterrückstand mit Alkohol und trocknet im Vakuum bei 70 °C. Man erhält 8.2 g eines gelben 1 : 2-Nickelkomplexes des Hydrazons der Formel

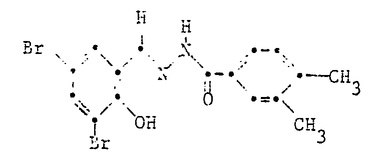

Mikroanalyse :
Berechnet: C 42.3 H 2.9 N 6.2 Ni 6.5 %
Gefunden : C 43.1 H 3.1 N 6.9 Ni 6.7 %.

### Beispiel 41

17.2 g 2-Hydroxy-1-naphthaldehyd und 13.6 g Benzhydrazid werden in 200 ml Wasser gegeben. Man setzt 20 ml Aethanol und 5 ml Essigsäure zu, erwärmt die Mischung auf 90 °C und rührt 90 Minuten bei dieser Temperatur. Man filtriert, wäscht mit Wasser und trocknet das Produkt bei 50 °C im Vakuum. Man erhält 28,4 g des Benzoylhydrazons der Formel

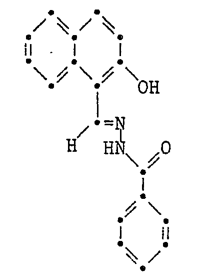

14,5 g dieses Benzoylhydrazons und 7 g Nickelacetattetrahydrat werden in 150 ml Wasser während 4 Stunden bei 90 °C gerührt. Das gelbe Produkt wird filtriert, mit Wasser gewaschen und bei 70 °C im Vakuum getrocknet. Man erhält 14,8 g eines gelben 1 : 2-Nickelkomplexes des Benzoylhydrazons der obigen Formel.

### Beispiel 42

10 g Titandioxyd und 2 g gemahlener 1 : 1-Kupferkomplex aus dem Benzoylhydrazon der Formel VII werden wie in Beispiel 2 beschrieben in einen Lack eingearbeitet, welcher auf Aluminiumfolie gespritzt. vorgetrocknet und eingebrannt wird. Man erhält eine kräftige, grünstichig gelbe Lackierung mit sehr guter Ueberlackier-, Licht- und Wetterechtheit sowie hohem Glanz.

### Beispiel 43

4 g gemahlener 1 : 1-Kupferkomplex aus dem Benzoylhydrazon der Formel VII und 76 g eines Lackansatzes, bestehend aus 41,3 % der 60 %-igen Lösung eines wärmehärtbaren Polyacrylates (Viacryl VC 373^{*} der Firma Vianova, Oesterreich) in Xylol, 16,3 % der 55 %-igen Lösung eines Melaminharzes (Maprenal TTX® der Cassella Farbwerke AG, BRD) in Butanol, 32,8 % Xylol, 4,6 % Aethylglykolacetat, 2 % Cyclohexanon, 2 % Butylacetat und 1 % einer 1 %-igen Siliconöl-Lösung in Xylol, werden 72 Stunden in der Kugelmühle dispergiert.

8 g dieser Mischung und 20 g einer 2 %-igen Aluminiumdispersion in Paraffin und Xylol werden gründlich vermischt und gleichmässig auf Blech gespritzt. Nach 30 Minuten Vortrocknen wird 30 Minuten bei 120-130 °C eingebrannt. Es entsteht eine kräftige gelb-olive Färbung mit sehr guter Wetterechtheit.

### Beispiele 44 bis 61

Weitere echte gelbe Ausfärbungen in Kunststoffen, Lacken, Fasern oder Druckfarben erhält man, wenn man analog zu den Beispielen 1-5 arbeitet, jedoch anstelle des dort verwendeten Pigmentes den 1 : 1-Metallkomplex eines in Tabelle 3 aufgeführten Hydrazons einsetzt.
(Siehe Tabelle 3 Seite 12 ff.)

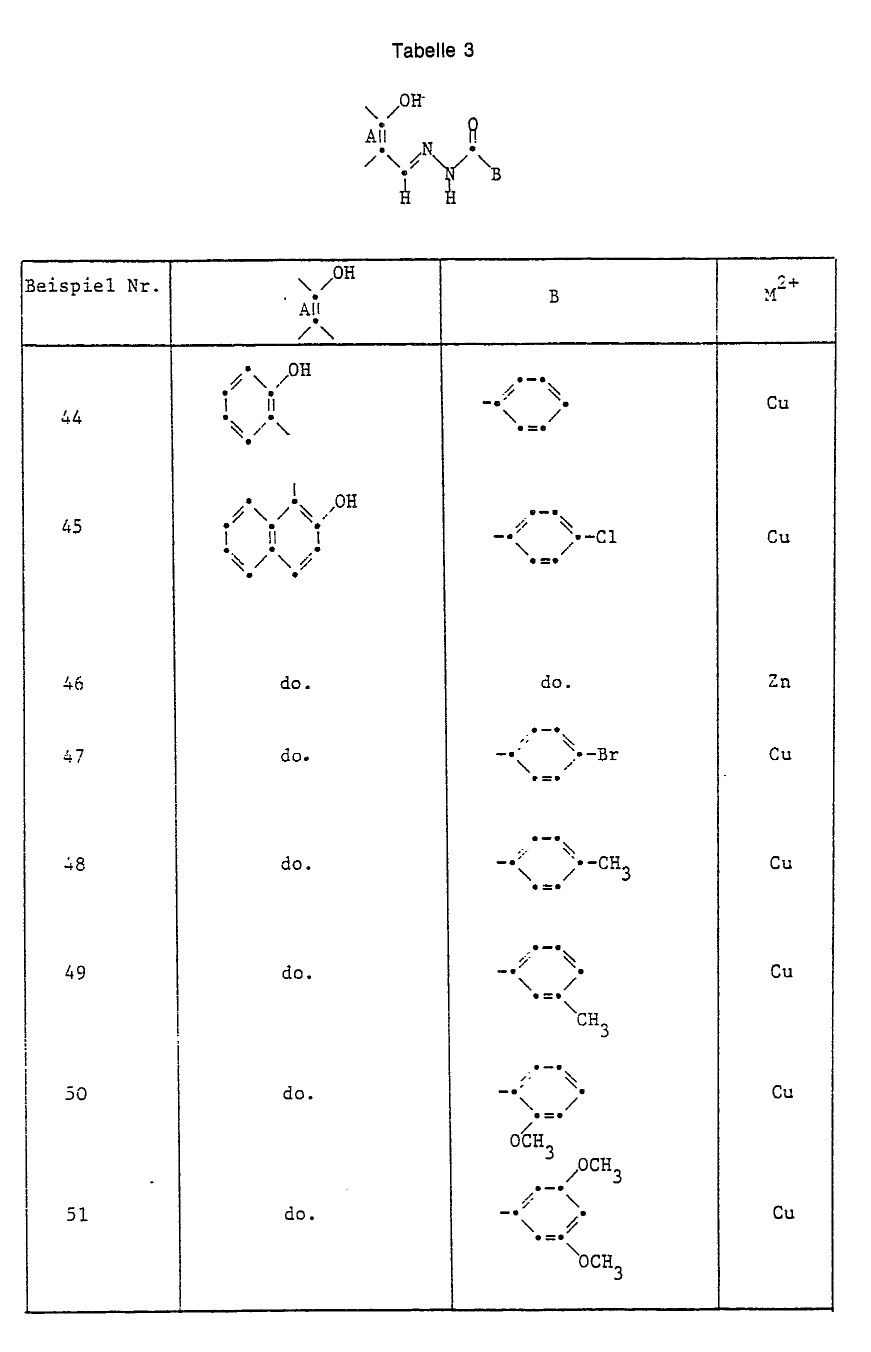

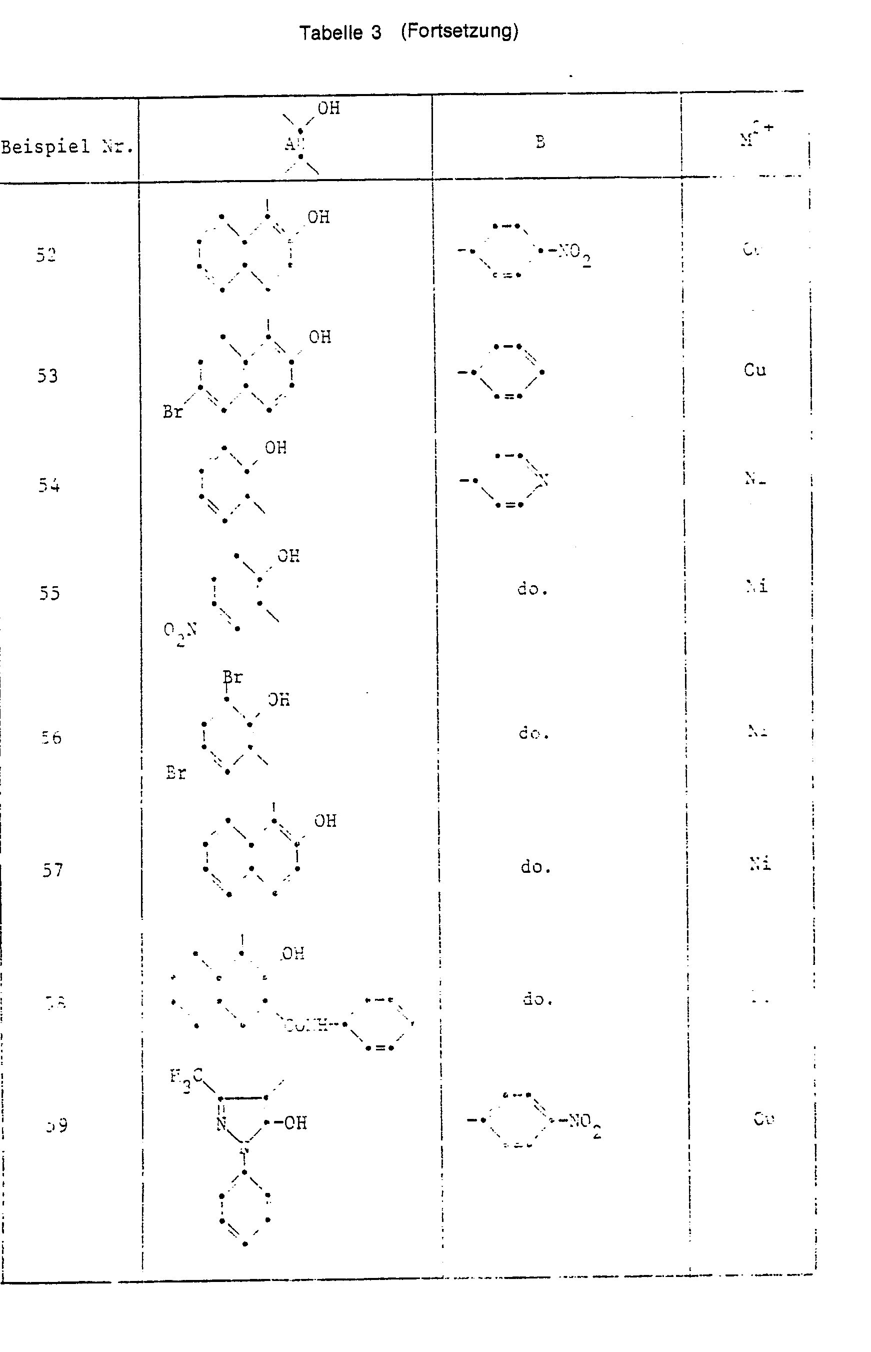

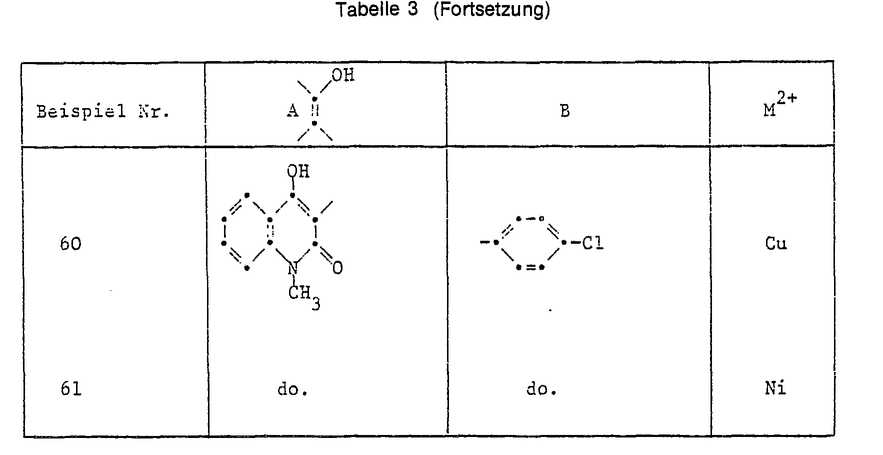

### Beispiele 62 bis 66

Weitere echte gelbe Ausfärbungen in Kunststoffen, Lacken, Fasern oder Druckfarben erhält man, wenn man analog zu den Beispielen 1-5 arbeitet, jedoch anstelle des dort verwendeten Pigmentes einen in Tabelle 4 aufgeführten 1 : 1-Metallkomplex einsetzt.

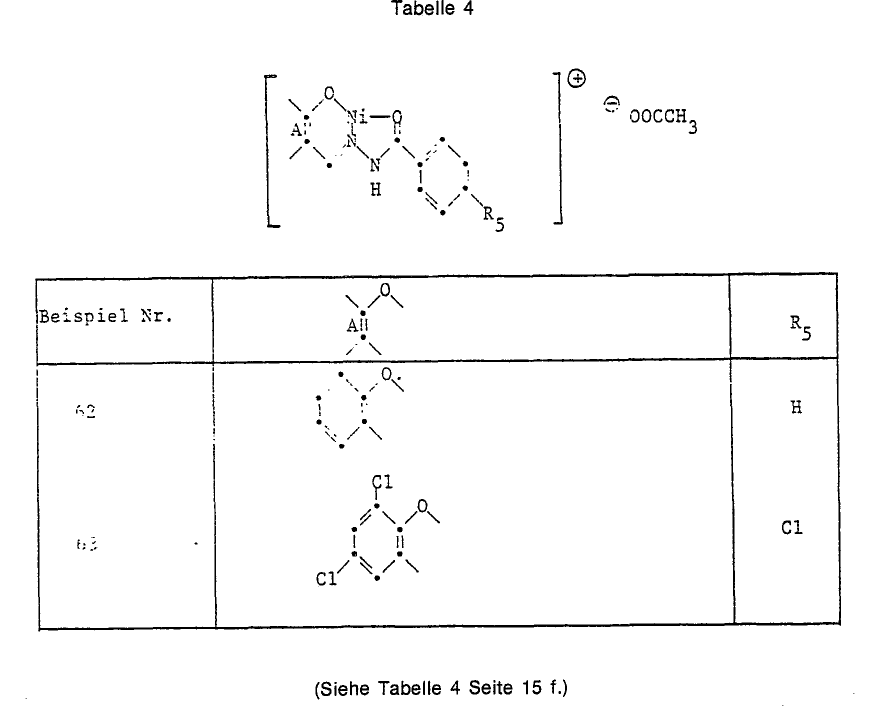

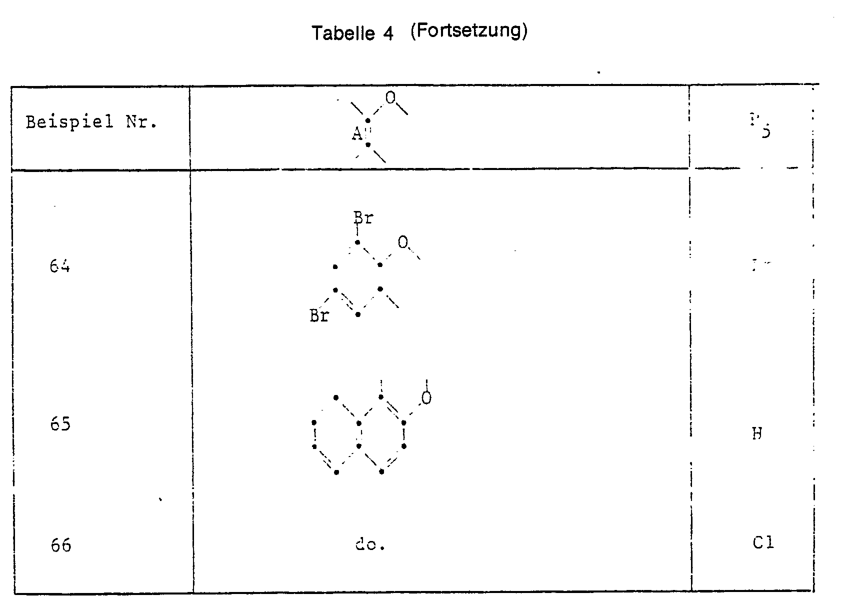

### Beispiel 67

3,4 g 2-Hydroxy-1-naphthaldehyd und 2,7 g Benzhydrazid werden in 30 ml Aethylcellosolve während 30 Minuten bei 100 °C kondensiert. Nach Zugabe von 5,2 g Nickelacetattetrahydrat wird die Mischung weitere 4 Stunden bei 120 °C gerührt. Ein gelbes Produkt fällt aus, das abfiltriert, mit Aethanol gewaschen und im Vakuum bei 80 °C getrocknet wird. Man erhält 7 g Kristallwasser enthaltendes Produkt der Formel

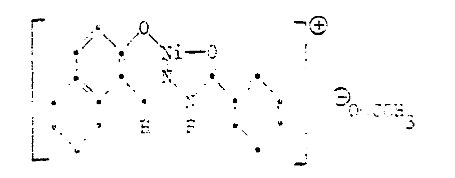

Mikroanalyse :
Berechnet : C 59,2 H 3,7 N 6,9 Ni 14,5 %
Gefunden : C 57,7 H 3,9 N 6,9 Ni 14,3 %.

### Beispiel 68

1,72 g 2-Hydroxy-1-naphthaldehyd und 1,36 g Benzhydrazid werden in 40 ml Aethylcellosolve 90 Minuten bei 100 °C kondensiert. Die Mischung wird nach Zugabe von 2,1 g Kupferacetatmonohydrat 31/2 Stunden bei 110 °C gerührt. Das ausgefallene gelbe Produkt wird bei 60 °C abfiltriert, mit Aethylcellosolve und Aethanol gewaschen und bei 80 °C im Vakuum getrocknet. Man erhält 3 g gelbes Pigment der Formel

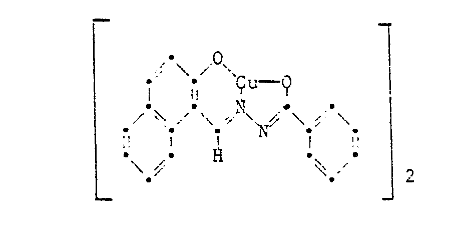

Mikroanalyse:
Berechnet: C 61,4 H 3,4 N 8,0 Cu 18,1 %
Gefunden : C 60,9 H 3,4 N 8,0 Cu 18,1 %.

## Patentansprüche

1. Verfahren zum Pigmentieren von hochmolekularem organischem Material, dadurch gekennzeichnet, dass man einen Uebergangsmetall-Komplex eines Hydrazons der Formel I verwendet 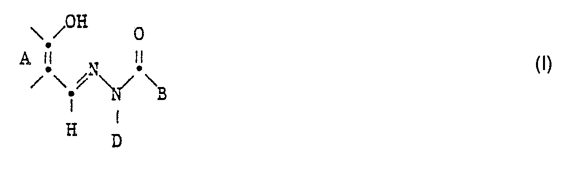 worin A einen isocyclischen oder heterocyclischen aromatischen Rest und B unsubstituiertes Phenyl oder Naphthyl oder durch ein bis zwei gleiche oder verschiedene Substituenten aus der Gruppe bestehend aus C₁-C₃ Alkyl, C₁-C₃ Alkoxy, Chlor, Brom, Nitro, Phenyl, Phenoxy, Chlorphenoxy, Bromphenoxy, Methylphenoxy, Methoxyphenoxy, Benzoylamino, Methylbenzoylamino, Methoxybenzoylamino, Chlorbenzoylamino und Brombenzoylamino substituiertem Phenyl oder Naphthyl bedeuten, und D Wasserstoff bedeutet, wobei, falls B ein Phenyl oder Naphthyl ist, D Wasserstoff oder eine Carbonylgruppe darstellt, die zusammen mit dem Strukturelement 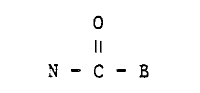 einen Phthalimid- oder Naphthalimidrest bildet. '

2. Verfahren gemäss Anspruch 1, dadurch gekennzeichnet, dass man den 1 : 2- oder 1 : 1-Nickelkomplex oder den 1 : 1-Kupferkomplex eines Hydrazons der Formel II verwendet, 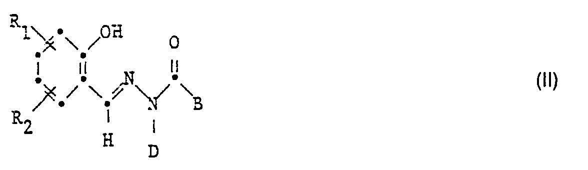 worin R₁ und R₂ unabhängig voneinander Wasserstoff, Chlor, Brom, C₁-C₃-Alkyl, C₁-C₃-Alkoxy, Cyano oder Nitro bedeuten, und B unsubstituiertes Phenyl oder Naphthyl oder durch ein bis zwei gleiche oder verschiedene Substituenten aus der Gruppe bestehend aus C₁-C₃-Alkyl C₁-C₃-Alkoxy, Chlor, Brom, Nitro, Phenyl, Phenoxy, Chlorphenoxy, Bromphenoxy, Methylphenoxy, Methoxyphenoxy, Benzoylamino, Methylbenzoylamino, Methoxybenzoylamino, Chlorbenzoylamino und Brombenzoylamino substituiertem Phenyl oder Naphthyl bedeutet, und D Wasserstoff oder eine Carbonylgruppe darstellt, die zusammen mit dem Strukturelement 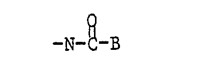 einen Phthalimid- oder Naphthalimidrest bildet.

3. Verfahren gemäss Anspruch 2, dadurch gekennzeichnet, dass man den 1 : 1- oder 1 : 2-Nickelkomplex oder den 1 : 1-Kupferkomplex eines Hydrazons der Formel II verwendet, worin R₁ und R₂ unabhängig voneinander Wasserstoff, Chlor oder Brom sind, und B unsubstituiertes Phenyl oder Naphthyl oder durch ein bis zwei gleiche oder verschiedene Substituenten aus der Gruppe bestehend aus Methyl, Methoxy, Chlor, Brom, Nitro oder Phenyl substituiertes Phenyl oder Naphthyl bedeutet, und D Wasserstoff ist.

4. Verfahren gemäss Anspruch 2, dadurch gekennzeichnet, dass man den 1 : 1- oder 1 2-Nickelkomplex oder 1 : 1-Kupferkomplex eines Hydrazons der Formel II verwendet, worin R₁ und R₂ Chlor sind, und B durch Methyl substituiertes Phenyl bedeutet, und D Wasserstoff ist.

5. Verfahren gemäss Anspruch 1, dadurch gekennzeichnet, dass man den 1 : 1- oder 1 :2-Nickelkomplex oder 1 : 1-Kupferkomplex eines Hydrazons der Formel III verwendet 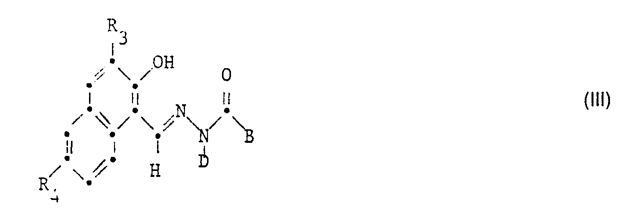 worin R₃ Wasserstoff, Carboxy, C₂-C₄-Alkoxycarbonyl, Carbamoyl, unsubstituiertes oder durch C₁-C₃-Alkyl, C₁-C₃-Alkoxy, Chlor oder Brom einfach substituiertes Phenylcarbamoyl bedeutet, und R₄ Wasserstoff, Chlor, Brom oder C₁-C₃-Alkoxy bedeutet, und B und D die in Patentanspruch 2 angegebene Bedeutung haben.

6. Verfahren gemäss Anspruch 5, dadurch gekennzeichnet, dass man den 1 : 1- oder 1 : 2-Nickelkomplex oder 1 : 1-Kupferkomplex eines Hydrazons der Formel III verwendet, worin R₃ Wasserstoff, Carboxy, Methoxycarbonyl, Carbamoyl oder Phenylcarbamoyl bedeutet, und R₄ Wasserstoff oder Brom ist, und B und D die in Patentanspruch 3 angegebene Bedeutung haben.

7. Verfahren gemäss Anspruch 5, dadurch gekennzeichnet, dass man den 1 : 1- oder 1 : 2-Nickelkomplex oder 1 : 1-Kupferkomplex eines Hydrazons der Formel III verwendet, worin R₃ Wasserstoff, Carboxy oder Phenylcarbamoyl bedeutet, und R₄ Wasserstoff oder Brom ist, und D Wasserstoff ist, und B unsubstituiertes oder durch Chlor, Brom, Methyl, Methoxy oder Nitro einfach substituiertes Phenyl bedeutet.

8. Verfahren gemäss Anspruch 5, dadurch gekennzeichnet, dass man den 1 : 1- oder 1 : 2-Nickelkomplex oder 1 : 1-Kupferkomplex eines Hydrazons der Formel III verwendet, worin R₃, R₄ und D Wasserstoff bedeuten und B unsubstituiertes Phenyl ist.

9. Verfahren gemäss Anspruch 2, dadurch gekennzeichnet, dass man den 1 : 2-Nickelkomplex eines Hydrazons der Formel II verwendet.

10. Verfahren gemäss Anspruch 5, dadurch gekennzeichnet, dass man den 1 2-Nickelkomplex eines Hydrazons der Formel III verwendet.

11. Verfahren gemäss Anspruch 2, dadurch gekennzeichnet, dass man den 1 : 1-Kupferkomplex eines Hydrazons der Formel II verwendet.

12. Verfahren gemäss Anspruch 5, dadurch gekennzeichnet, dass man den 1 : 1-Kupferkomplex eines Hydrazons der Formel III verwendet.

13. 1 : 1-, 1 2-Nickelkomplexe und 1 : 1-Kupferkomplexe von Hydrazonen der Formel I gemäss Anspruch 1, worin A einen heterocyclischen aromatischen Rest darstellt, und die Symbole B und D die in Anspruch 1 angegebene Bedeutung haben, und von Hydrazonen der Formel II gemäss Anspruch 2 und der Formel III gemäss Anspruch 5, worin R₁ und R₂ beide entweder Chlor oder Brom sind, R₃ Wasserstoff, Carboxy, C₂-C₇-Alkoxycarbonyl, Carbamoyl oder Phenylcarbamoyl bedeutet, R₄ Wasserstoff oder Brom ist, B unsubstituiertes Phenyl oder Naphthyl oder durch C₁-C₆-Alkyl, C₁-C₆-Alkoxy, Halogen, Nitro, Phenyl, Phenoxy, Halogenphenoxy, C₁-C₁₂-Alkylphenoxy, C₁-C₁₂-Alkoxyphenoxy, Benzoylamino, C₈-C₁₃-Alkylbenzoylamino, C₈-C₁₃-Alkoxybenzoylamino, Halogenbenzoylamino substituiertes Phenyl oder Naphthyl bedeutet, und D Wasserstoff oder eine Carbonylgruppe ist, die zusammen mit dem Strukturelement 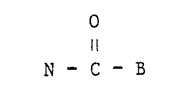 einen Phthalimid- oder Naphthalimidrest bildet, wobei R₃ oder R₄ verschieden von Wasserstoff sein muss, wenn D Wasserstoff ist und B unsubstituiertes Phenyl bedeutet.

14. Verfahren zur Herstellung von Uebergangsmetallkomplexen von Hydrazonen der Formel I gemäss Anspruch 1, dadurch gekennzeichnet, dass man eine Verbindung der Formel IV 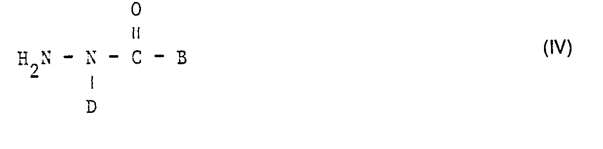 worin D'und B die in Patentanspruch 1 angegebene Bedeutung haben, mit einem Aldehyd der Formel V 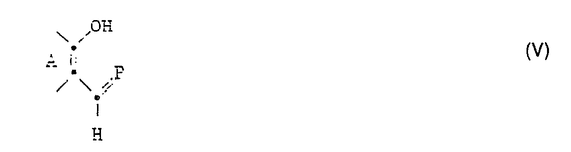 oder einem Aldimin der Formel VI 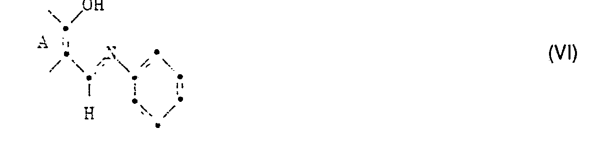 worin A die in Patentanspruch 1 angegebene Bedeutung hat, in Wasser als Lösungsmittel kondensiert und während oder nach der Kondensation ein metallabgebendes Mittel zusetzt.

15. Hochmolekulares organisches Material, enthaltend einen Uebergangsmetall-Komplex eines Hydrazons der Formel I nach Anspruch 1.

## Claims

1. A process for pigmenting high molecular organic material, which process comprises the use of a transition metal complex of a hydrazone of the formula I 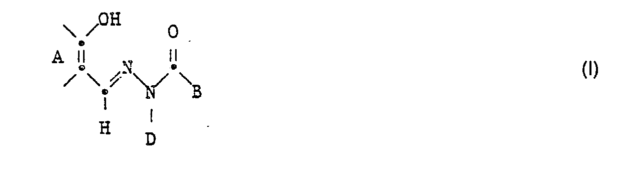 wherein A is an isocyclic or heterocyclic radical and B is unsubstituted phenyl or naphthyl, or phenyl or naphthyl each substituted by one or two identical or different substituents selected from the group consisting of C₁-C₃-alkyl, C₁-C₃-alkoxy, chlorine, bromine, nitro, phenyl, phenoxy, chlorophenoxy, bromophenoxy, methylphenoxy, methoxyphenoxy, benzoylamino, methylbenzoylamino, methoxybenzoylamino, chlorobenzoylamino and bromobenzoylamino, and D is hydrogen, with the proviso that if B is a phenyl or naphthyl group, D is hydrogen, or a carbonyl group which together with the structural element 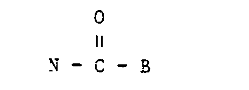 forms a phthalimide or naphthalimide group.

2. A process according to claim 1, which comprises the use of the 1 : 2- or 1 : 1-nickel complex or the 1 : 1-copper complex of a hydrazone of the formula II 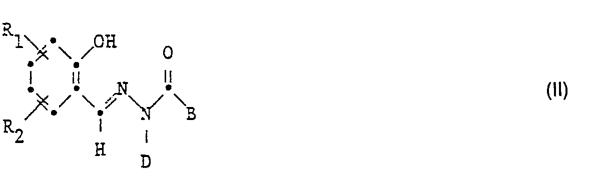 wherein each of R₁ and R₂ independently of the other is hydrogen, chlorine, bromine, C₁-C₃-alkyl, C₁-C₃- alkoxy, cyano or nitro ; B is unsubstituted phenyl or naphthyl, or phenyl or naphthyl which are each substituted by one or two identical or different substituents selected from the group consisting of Cₗ-C₃- alkyl, C₁-C₃-alkoxy, chlorine, bromine, nitro, phenyl, phenoxy, chlorophenoxy, bromophenoxy, methylphenoxy, methoxyphenoxy, benzoylamino, methylbenzoylamino, methoxybenzoylamino, chlorobenzoylamino and bromobenzoylamino ; and D is hydrogen, or a carbonyl group which together with the structural element 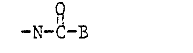 forms a phthalimide or naphthalimide group.

3. A process according to claim 2, which comprises the use of the 1 : 1- or 1 : 2-nickel complex or the 1 : 1-copper complex of a hydrazone of the formula II in which each of R, and R₂ independently of the other is hydrogen, chlorine or bromine ; B is unsubstituted phenyl or naphthyl, or phenyl or naphthyl each substituted by one or two identical or different substituents selected from the group consisting of methyl, methoxy, chlorine, bromine, nitro or phenyl ; and D is hydrogen.

4. A process according to claim 2, which comprises the use of the 1 : 1- or 1 : 2-nickel complex or 1 : 1-copper complex of a hydrazone of the formula II in which R₁ and R₂ are chlorine ; B is phenyl substituted by methyl ; and D is hydrogen.

5. A process according to claim 1, which comprises the use of the 1 : 1- or 1 : 2-nickel complex or 1 : 1-copper complex of a hydrazone of the formula III 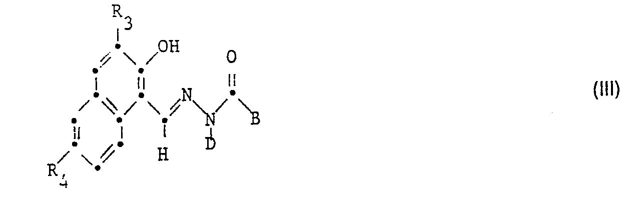 in which R₃ is hydrogen, carboxyl, C₂-C₄-alkoxycarbonyl, carbamoyl, or phenylcarbamoyl which is unsubstituted or monosubstituted by C₁-C₃-alkyl, C₁-C₃-alkoxy, chlorine or bromine, and R₄ is hydrogen, chloride, bromine or C₁-C₃-alkoxy ; and B and D are as defined in claim 2.

6. A process according to claim 5, which comprises the use of the 1 : 1- or 1 : 2-nickel complex or 1 1-copper complex of a hydrazone of the formula III in which R₃ is hydrogen, carboxyl, methoxycarbonyl, carbamoyl or phenylcarbamoyl ; R₄ is hydrogen or bromine ; and B and D are as defined in claim 3.

7. A process according to claim 5, which comprises the use of the 1 : 1- or 1 : 2-nickel complex or 1 1-copper complex of a hydrazone of the formula III in which R₃ is hydrogen, carboxyl or phenylcarbamoyl ; R₄ is hydrogen or bromine ; D is hydrogen ; and B is phenyl which is unsubstituted or monosubstituted by chlorine, bromine, methyl, methoxy or nitro.

8. A process according to claim 5, which comprises the use of the 1 : 1- or 1 : 2-nickel complex of 1 : 1-copper complex of a hydrazone of the formula III in which R₃, R₄ and D are hydrogen ; and B is unsubstituted phenyl.

9. A process according to claim 2, which comprises the use of the 1 2-nickel complex of a hydrazone of the formula II.

10. A process according to claim 5, which comprises the use of the 1 : 2-nickel complex of a hydrazone of the formula III.

11. A process according to claim 2, which comprises the use of the 1 : 1-copper complex of a hydrazone of the formula II.

12. A process according to claim 5, which comprises the use of the 1 : 1-copper complex of a hydrazone of the formula III.

13. A 1 : 1-, 1 : 2-nickel complex of 1 : 1-copper complex of a hydrazone of the formula I according to claim 1 wherein A is a heterocyclic aromatic radical, and the symbols B and D are as defined in claim 1 : and of a hydrazone of the formula II according to claim 2, and of the formula III according to claim 5, in which R₁ and R₂ are both either chlorine or bromine, R₃ is hydrogen, carboxyl, C₂-C₇-alkoxycarbonyl, carbamoyl or phenylcarbamoyl, R₄ is hydrogen or bromine, B is unsubstituted phenyl or naphthyl, or phenyl or naphthyl each substituted by C₁-C₆-alkyl, C₁-C₆-alkoxy, halogen, nitro, phenyl, phenoxy, halophenoxy, C₇-C₁₂-alkylphenoxy, C₇-C₁₂-alkoxyphenoxy, benzoylamino, C₈-C₁₃-alkylbenzoylamino, C₈-C₁₃-alkoxybenzoylamino or halobenzoylamino, and D is hydrogen, or a carbonyl group which together with the structural element 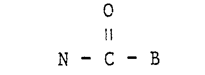 forms a phthalimide or naphthalimide group, with the proviso that R₃ or R₄ must have a meaning other than hydrogen when D is hydrogen, and B is unsubstituted phenyl.

14. A process for the preparation of a transition metal complex of a hydrazone of the formula I according to claim 1, which process comprises condensing a compound of the formula IV 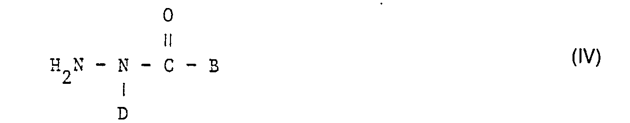 wherein D and B are as defined in claim 1, with an aldehyde of the formula V 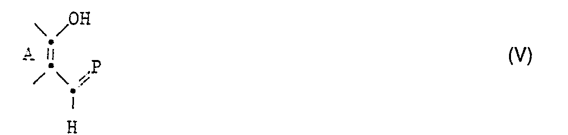 or with an aldimine of the formula VI 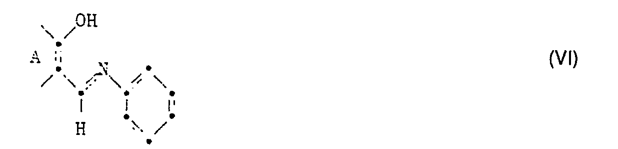 in which A is as defined in claim 1, in water as the solvent ; and adding a metal donor either during or after the condensation reaction.

15. Highmolecular organic material containing a transition metal complex of a hydrazone of the formula I according to claim 1.

## Revendications

1. Procédé pour pigmenter des matières organiques macromoléculaires, procédé caractérisé en ce qu'on utilise un complexe d'un métal de transition d'une hydrazone répondant à la formule 1 : 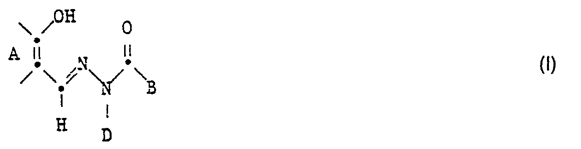 dans laquelle A représente un radical aromatique isocyclique ou hétérocyclique, B représente un radical phényle ou naphtyle non substitué ou un radical phényle ou naphtyle portant un substituant ou deux substituants, identiques ou différents, pris dans l'ensemble constitué par les alkyles en C,-C₃, les alcoxy en C,-C₃, le chlore, le brome, le nitro, le phényle, le phénoxy, les chlorophénoxy, les bromophénoxy, les méthylphénoxy, les méthoxyphénoxy, les benzoylamino, les méthylbenzoylamino, les méthoxybenzoylamino, les chlorobenzoylamino et les bromobenzoylamino, et D représente l'hydrogène et peut aussi, dans le cas où B désigne un radical phényle ou naphtyle, représenter un radical carbonyle formant, avec l'élément structural : 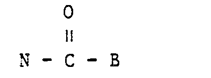 un radical de phtalimide ou de naphtalimide.

2. Procédé selon la revendication 1 caractérisé en ce qu'on utilise le complexe de nickel 1 : 2 ou 1 : 1 ou le complexe de cuivre 1 : 1 d'une hydrazone répondant à la formule II : 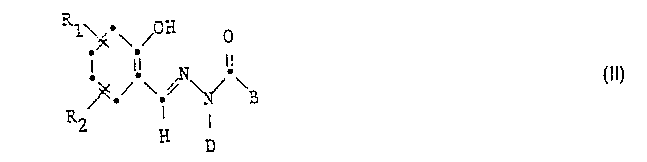 dans laquelle R₁ et R₂ représentent chacun, indépendamment l'un de l'autre, l'hydrogéne, le chlore, le brome, un alkyle en C₁-C₃, un alcoxy en C₁-C₃, un cyano ou un nitro, B représente un radical phényle ou naphtyle non substitué ou un radical phényle ou naphtyle portant 1 substituant ou 2 substituants. identiques ou différents, pris dans l'ensemble constitué par les alkyles en C₁-C₃, les alcoxy en C₁-C₃, le chlore, le brome, le nitro, le phényle, le phénoxy, les chlorophénoxy, les bromophénoxy, les méthylphénoxy, les méthoxyphénoxy, le benzoylamino, les méthylbenzoylamino, les méthoxybenzoylamino, les chlorobenzoylamino et les bromobenzoylamino, et D représente l'hydrogène ou un radical carbonyle formant, avec l'élément structural 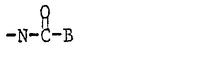 un radical de phtalimide ou de naphtalimide.

3. Procédé selon la revendication 2 caractérisé en ce qu'on utilise le complexe de nickel 1 : 1 ou 1 : 2 ou le complexe de cuivre 1 : 1 d'une hydrazone de formule Il dans laquelle R, et R₂ représentent chacun. indépendamment l'un de l'autre, l'hydrogène, le chlore ou le brome, B représente un radical phényle ou naphtyle non substitué ou un radical phényle ou naphtyle portant 1 substituant ou 2 substituants, identiques ou différents, pris dans l'ensemble constitué par le méthyle, le méthoxy, le chlore, le brome, le nitro et le phényle, et D représente l'hydrogène.

4. Procédé selon la revendication 2 caractérisé en ce qu'on utilise le complexe de nickel 1 : 1 ou 1 : 2 ou le complexe de cuivre 1 : 1 d'une hydrazone de formule Il dans laquelle R, et R₂ représentent chacun le chlore, B représente un radical phényle porteur d'un méthyl, et D représente l'hydrogène.

5. Procédé selon la revendication 1 caractérisé en ce qu'on utilise le complexe de nickel 1 : 1 ou 1 : 2 ou le complexe de cuivre 1 : 1 d'une hydrazone répondant à la formule III : 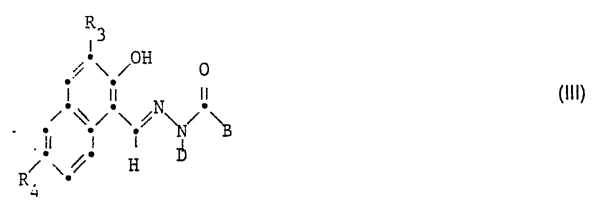 dans laquelle R₃ représente l'hydrogène, un carbnxy, un alcoxycarbonyle en C₂-C₄, un carbamoyle, un phénylcarbamoyle non substitué ou un phénylcarbamoyle monosubstitué dont le substituant est un alkyle en C₁-C₃, un alcoxy en C₁-C₃, le chlore ou le brome, R₄ représente l'hydrogène, le chlore, le brome ou un alcoxy en C₁-C₃, et B et D ont les significations qui leur ont été données à la revendication 2.

6. Procédé selon la revendication 5 caractérisé en ce qu'on utilise le complexe de nickel 1 : 1 ou 1 : 2 ou le complexe de cuivre 1 : 1 d'une hydrazone de formule III dans laquelle R₃ représente l'hydrogène, un carboxy, un méthoxycarbonyle, un carbamoyle ou un phénylcarbamoyle, R₄ représente l'hydrogène ou le brome, et B et D ont les significations données à la revendication 3.

7. Procédé selon la revendication 5 caractérisé en ce qu'on utilise le complexe de nickel 1 : 1 ou 1 : 2 ou le complexe de cuivre 1 : 1 d'une hydrazone de formule III dans laquelle R₃ représente l'hydrogène, un carboxy ou un phénylcarbamoyle, R₄ l'hydrogène ou le brome, D l'hydrogène et B un radical phényle non substitué ou porteur d'1 substituant, celui-ci étant le chlore, le brome, un méthyle, un méthoxy ou un nitro.

8. Procédé selon la revendication 5 caractérisé en ce qu'on utilise le complexe de nickel 1 : 1 ou 1 : 2 ou le complexe de cuivre 1 : 1 d'une hydrazone de formule III dans laquelle R₃, R₄ et D représentent chacun l'hydrogène et B représente un phényle non substitué.

9. Procédé selon la revendication 2 caractérisé en ce qu'on utilise le complexe de nickel 1 : 2 d'une hydrazone de formule II.

10. Procédé selon la revendication 5 caractérisé en ce qu'on utilise le complexe du nickel 1 : 2 d'une hydrazone de formule III.

11. Procédé selon la revendication 2 caractérisé en ce qu'on utilise le complexe de cuivre 1 : 1 d'une hydrazone de formule II.

12. Procédé selon la revendication 5 caractérisé en ce qu'on utilise le complexe de cuivre 1 : 1 d'une hydrazone de formule III.

13. Complexes de nickel 1 : 1 et 1 : 2 et complexes de cuivre 1 : 1 d'hydrazones de formule 1 selon la revendication 1 dans lesquelles A représente un radical hétérocyclique aromatique et les symboles B et D ont les significations données à la revendication 1, et d'hydrazones de formule Il selon la revendication 2 et de formule III selon la revendication 5 dans lesquelles R₁ et R₂ représentent soit chacun le chlore soit chacun le brome, R₃ représente l'hydrogène, un carboxy, un alcoxycarbonyle en C₂-C₇, un carbamoyle ou un phénylcarbamoyle. R₄ représente l'hydrogène ou le brome. B représente un radical phényle ou naphtyle non substitué ou un radical phényle ou naphtyle porteur d'un alkyle en C₁-C₆, d'un alcoxy en C₁-C₆, d'un halogène, d'un nitro, d'un phényle, d'un phénoxy, d'un halogénophénoxy, d'un alkylphénoxy en C₇-C₁₂, d'un alcoxyphénoxy en C₇-C₁₂, d'un benzoylamino, d'un alkylbenzoylamino en C₈-C₁₃, d'un alcoxybenzoylamino en C₈-C₁₃ ou d'un halogénobenzoylamino, et D représente l'hydrogène ou un radical carbonyle formant, avec l'élément structural de formule : 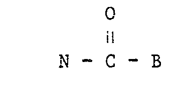 un radical de phtalimide ou de naphtalimide avec la condition que R3 ou R₄ ne doit pas être l'hydrogène dans le cas où D représente l'hydrogène et B un phényle non substitué.

14. Procédé de préparation de complexes de métaux de transition d'hydrazones de formule I selon la revendication 1, procédé caractérisé en ce qu'on condense, en opérant dans un solvant qui est l'eau, un composé répondant à la formule IV : 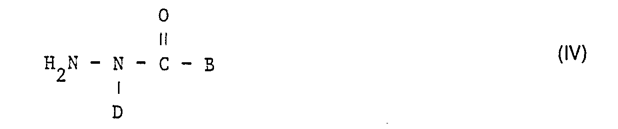 dans laquelle D et B ont les significations données à la revendication 1, avec un aldéhyde de formule V : 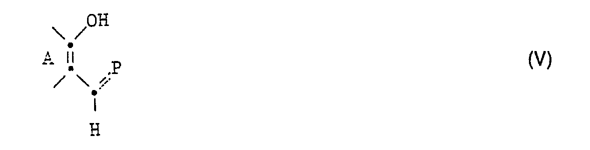 ou une aldimine de formule VI : 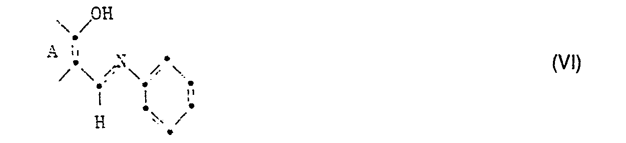 formules dans lesquelles A a la signification donnée à la revendication 1, et, pendant ou après la condensation, on ajoute un agent capable de céder un métal.

15. Matières organiques macromoléculaires qui contiennent un complexe de métal de transition d'une hydrazone de formule I selon la revendication 1.
